# EUROPÄISCHE PATENTSCHRIFT

(11) **EP 0 768 343 B1**
(45) Veröffentlichungstag und Bekanntmachung des Hinweises auf die Patenterteilung: **21.02.2001**
(21) Anmeldenummer: 96115892.0
(22) Anmeldetag: 04.10.1996
(51) Int. Cl.: C09C 1/00, C09D 7/12, C09D 11/00, C08K 9/02, C03C 4/02, C04B 33/14, A61K 7/00

(54) **Goniochromatische Glanzpigmente mit siliciumhaltiger Beschichtung**
Brilliant goniochromatic pigments with a silicon containing coating
Pigments brillants goniochromatiques à revêtement contenant du silicium

(30) Priorität: 14.10.1995 DE 19538295
(43) Veröffentlichungstag der Anmeldung: 16.04.1997
(73) Patentinhaber: BASF Aktiengesellschaft, 67063 Ludwigshafen (DE)
(72) Erfinder: Schmid, Raimund, Dr., 67435 Neustadt (DE); Mronga, Norbert, Dr., 69221 Dossenheim (DE); Adel, Jörg, Dr., 67071 Ludwigshafen (DE)

(56) Entgegenhaltungen:
- EP-A- 0 571 836
- EP-A- 0 668 329

## Beschreibung

Die vorliegende Erfindung betrifft neue goniochromatische Glanzpigmente auf der Basis von mehrfach beschichteten, plättchenförmigen, metallischen Substraten, die mindestens ein Schichtpaket aus
A) einer im wesentlichen aus Siliciumoxid, Siliciumoxidhydrat, Aluminiumoxid und/oder Aluminiumoxidhydrat bestehenden Schicht und
B) einer nichtselektiv absorbierenden, für sichtbares Licht zumindest teilweise durchlässigen, elementares Silicium enthaltenden Schicht
sowie gewünschtenfalls zusätzlich
C) eine äußere Schicht, die im wesentlichen aus farblosem oder selektiv absorbierendem Metalloxid besteht,
aufweisen.

Weiterhin betrifft die Erfindung Mischungen aus den Pigmenten (I) und mehrfach beschichteten silikatischen Plättchen (II), die
A') eine im wesentlichen aus farblosem oder selektiv absorbierendem Metalloxid bestehende Schicht,
B') eine nichtselektiv absorbierende, metallische, für sichtbares Licht zumindest teilweise durchlässige, elementares Silicium enthaltende Schicht und gewünschtenfalls
C') eine äußere Schicht, die im wesentlichen aus farblosem oder selektiv absorbierendem Metalloxid besteht,
aufweisen, als wesentlichen Komponenten.

Außerdem betrifft die Erfindung die Herstellung der goniochromatischen Glanzpigmente und ihre Verwendung zum Einfärben von Lacken, Druckfarben, Tinten, Kunststoffen, Gläsern, keramischen Produkten und Zubereitungen der dekorativen Kosmetik. Glanz- oder Effektpigmente werden in vielen Bereichen der Technik eingesetzt, beispielsweise in Automobillacken, in der dekorativen Beschichtung, der Kunststoffeinfärbung, in Anstrich-, Druck-, insbesondere Sicherheitsdruckfarben sowie in der Kosmetik.

Ihre optische Wirkung beruht auf der gerichteten Reflexion von Licht an überwiegend flächig ausgebildeten, zueinander parallel ausgerichteten, metallischen oder stark lichtbrechenden Pigmentteilchen. Je nach Zusammensetzung der Pigmentplättchen erzeugen Interferenz-, Reflexions- und Absorptionsphänomene winkelabhängige Farb- und Helligkeitseindrücke.

Aufgrund ihrer nicht kopierbaren optischen Effekte gewinnen diese Pigmente zunehmende Bedeutung für die Herstellung von fälschungssicheren Wertschriften, wie Geldscheinen, Schecks, Scheckkarten, Kreditkarten, Steuermarken, Briefmarken, Bahn- und Flugtickets, Telefonkarten, Lotterielosen, Geschenkzertifikaten, Ausweisen und Identifikationskarten.

Kennzeichnungen, die mit den Effektpigmenten angefertigt wurden, und das Fehlen dieser Kennzeichnungen oder ihre Veränderung, beispielsweise in einer Farbkopie (Verschwinden von Farbflops und Glanzeffekten), sind ohne Hilfsmittel mit bloßem Auge sicher erkennbar und ermöglichen so eine leichte Unterscheidung des Originals von der Kopie.

Glanzpigmente auf Basis metallischer Substrate sind aufgrund ihres guten Deckvermögens auch für Automobillackierungen von besonderem Interesse.

Bislang werden hierfür vor allem eisenoxidbeschichtete Aluminiumpigmente, wie sie in der EP-A-33 457 beschrieben werden, eingesetzt, die im Glanzwinkel intensive, goldene bis rote Absorptionsfarben zeigen, bei steileren Betrachtungswinkeln jedoch unbunt wirken. Um Lackierungen mit einem sogenannten two-tone Effekt zu erzielen, werden diese Pigmente daher mit Buntpigmenten anderer Farbe gemischt.

Goniochromatische Pigmente, die einen winkelabhängigen Farbwechsel zwischen unterschiedlichen Interferenzfarben zeigen, also bereits ohne Zusatz weiterer Pigmente in Lackierungen oder Drucken einen two-tone Effekt bewirken, sind z.B. aus der US-A-3 438 796 bekannt, in der symmetrisch aufgebaute Pigmente beschrieben werden, die aus einem zentralen opaken Aluminiumfilm (60 nm dick) bestehen, der beidseitig jeweils mit einem dickeren SiO₂-Film (500 nm), einem transparenten Aluminiumfilm (20 nm) und einem dünneren SiO₂-Film (200 nm) beschichtet ist.

Diese Pigmente werden hergestellt, indem eine Substratfolie im Hochvakuum abwechselnd mit SiO₂ und Aluminium bedampft wird. Der auf diese Weise aufgedampfte mehrschichtige Film wird anschließend von der Folie entfernt, z.B. abgekratzt, und auf für Glanzpigmente typische Teilchengrößen (etwa 5-50 µm) zerkleinert.

Durch die Art der Herstellung bedingt, ist der zentrale Metallfilm dieser Pigmente nur an der Plättchenober- und -unterseite beschichtet und daher nur unvollständig vor Umwelteinflüssen oder dem Angriff von Chemikalien geschützt. Zudem ist das Herstellungsverfahren auch sehr aufwendig und kostspielig.

In der US-A-4 879 140 wird ein Verfahren beschrieben, bei dem verdampfte Metallverbindungen in der Gasphase unter Einwirkung eines Mikrowellenplasmas zersetzt werden und die Zersetzungsprodukte filmartig auf der Reaktorwand abgeschieden werden. Siliciumfilme werden z.B. durch Zersetzung von SiH₄ und SiO₂-Filme durch Zersetzung von Siliciumtetrachlorid in Gegenwart von Sauerstoff erhalten. Durch eine Reihe von Maßnahmen können die abgeschiedenen Filme auf Pigmentgröße zerkleinert werden. Die so erhältlichen Pigmente weisen jedoch ebenfalls die obengenannten Nachteile auf.

Weiterhin sind aus der DE-A-44 05 492 goniochromatische Glanzpigmente bekannt, bei denen Aluminiumplättchen zunächst naßchemisch durch hydrolytische Zersetzung siliciumorganischer Verbindungen mit Siliciumoxid und anschließend durch Gasphasenbeschichtung (chemical vapor deposition, CVD) mit metallischen Schichten, wie molybdän-, eisen- oder chromhaltigen Schichten, belegt werden. In der Regel ist zur Erhöhung der Echtheiten dieser Pigmente, insbesondere der Schwitzwasserbeständigkeit, eine Belegung mit einer passivierenden Schutzschicht erforderlich. In der nicht vorveröffentlichten DE-A-44 19 173 werden außerdem magnetisierbare Glanzpigmente beschrieben, bei denen die Aluminiumplättchen zusätzlich mit einer inneren ferromagnetischen Schicht belegt sind. Aus der älteren deutschen Patentanmeldung 19516181.5 sind ähnliche Glanzpigmente bekannt, die eine im wesentlichen aus Aluminium bestehende metallische Beschichtung aufweisen.

Aus der nicht vorveröffentlichten DE-A-44 37 752 ist ein CVD-Verfahren bekannt, nach dem die SiO₂-Schicht auch durch Gasphasenbeschichtung von Siliciumorganylen aufgebracht werden kann.

Der Erfindung lag die Aufgabe zugrunde, goniochromatische Glanzpigmente ohne die genannten Nachteile und mit vorteilhaften Anwendungseigenschaften bereitzustellen.

Demgemäß wurden die eingangs definierten Glanzpigmente und ihre Mischungen mit mehrfach beschichteten silikatischen Plättchen gefunden.

Weiterhin wurde ein Verfahren zur Herstellung dieser Glanzpigmente gefunden, welches dadurch gekennzeichnet ist, daß man
die Schichten (A) durch Gasphasenzersetzung flüchtiger Siliciumorganyle mit Wasserdampf und/oder Sauerstoff oder durch hydrolytische Zersetzung organischer Silicium- oder Aluminiumverbindungen, bei denen die organischen Reste über Sauerstoffatome an das Metall gebunden sind, in Gegenwart eines organischen Lösungsmittels, in welchem die Metallverbindungen löslich sind und welches mit Wasser mischbar ist, und anschließende Trocknung,
die Schichten (B) durch Gasphasenzersetzung von Silanen der Formel I

SiₙX₂ₙ₊₂ I

in der die Variablen folgende Bedeutung haben:
- n: 1 bis 3;
- X: für den Fall n=1 Wasserstoff oder gleiche oder verschiedene C₁-C₃-Alkylreste, wobei mindestens ein Rest X Wasserstoff bedeutet, und
für den Fall n>1 Wasserstoff,
und gegebenenfalls Metallcarbonylen in einer inerten Atmosphäre und gewünschtenfalls
die Schicht (C) durch Gasphasenzersetzung flüchtiger Metallverbindungen in Gegenwart von Sauerstoff und/oder Wasserdampf
auf die metallischen Substratplättchen aufbringt.

Schließlich wurde die Verwendung dieser Glanzpigmente und Glanzpigmentmischungen zur Einfärbung von Lacken, Druckfarben, Tinten, Kunststoffen, Gläsern, keramischen Produkten und Zubereitungen der dekorativen Kosmetik gefunden.

Für die erfindungsgemäßen Glanzpigmente sind als Substrat alle für Metalleffektpigmente bekannten Metalle und Legierungen in Plättchenform geeignet. Z.B. kommen neben Stahl, Kupfer und seinen Legierungen wie Messing und Bronzen vor allem Aluminium und seine Legierungen wie Aluminiumbronze in Betracht.

Bevorzugt sind Aluminiumflakes, die in einfacher Weise durch Herausstanzen aus Aluminiumfolie oder nach gängigen Verdüsungs- und Mahltechniken herzustellen sind.

So sind beispielsweise Aluminiumplättchen geeignet, die nach dem sogenannten Hall-Verfahren in Testbenzin durch Naßmahlung hergestellt werden. Ausgangsmaterial ist ein atomisierter, spratziger Aluminiumgrieß, welcher in Kugelmühlen in Testbenzin und in Gegenwart eines Schmiermittels zu plättchenförmigen Teilchen verformt bzw. zerkleinert und anschließend klassiert wird.

Es können handelsübliche Produkte eingesetzt werden. Jedoch sollte die Oberfläche der Aluminiumteilchen weitgehend frei von Fetten oder anderen Belegmitteln sein. Diese Substanzen können zum Teil durch Lösungsmittelbehandlung oder besser, wie in der DE-A-42 23 384 beschrieben, durch oxidative Behandlung entfernt werden.

Weiterhin können die metallischen Substratteilchen passiviert sein, d.h. insbesondere gegenüber Wasser stabilisierende Beschichtungen aufweisen, wie sie z.B. aus der DE-A-42 36 332 und der nicht vorveröffentlichten DE-A-44 14 079 bekannt sind.

Als passivierende Beschichtungen sollen dabei auch Metalloxidschichten verstanden werden. Beispiele für weitere geeignete Substrate sind daher eisenoxidbeschichtete Metallpigmente (z.B. EP-A-33 457) mit (schwacher) goldener bis roter Eigenfarbe und zart pastellfarbene titandioxidbeschichtete Metallpigmente (z.B. EP-A-338 428). Die Metalloxidschicht sollte jedoch nicht zu dick sein, damit die Substratteilchen ihre "metallische Koloristik" behalten.

Schließlich sind auch magnetisierbare Aluminiumplättchen als Substratmaterial geeignet, die eine ferromagnetische, Eisen, Cobalt, Nickel, Magnetit oder γ-Fe₂O₃ enthaltende Beschichtung aufweisen (DE-A-43 13 541, DE-A-43 40 141 und die nicht vorveröffentlichte DE-A-44 19 173) und die Herstellung magnetisierbarer goniochromatischer Glanzpigmente ermöglichen.

Die Größe der Substratteilchen ist an sich nicht kritisch und kann auf den jeweiligen Anwendungszweck abgestimmt werden. In der Regel haben die Teilchen mittlere größte Durchmesser von etwa 1 bis 200 µm, insbesondere etwa 5 bis 100 µm, und Dicken von etwa 0,1 bis 5 µm, insbesondere um etwa 0,5 µm. Ihre spezifische freie Oberfläche (BET) beträgt im allgemeinen 0,1 bis 5 m²/g.

Die erfindungsgemäßen Glanzpigmente zeichnen sich durch eine Mehrfachbeschichtung des metallischen Substrats aus.

Die Schicht (A) enthält als wesentliche Bestandteile Aluminiumoxid, Aluminiumoxidhydrat und bevorzugt Siliciumoxid und Siliciumoxidhydrat sowie auch deren Mischungen.

Die Dicke der Schicht (A) beträgt im allgemeinen 20 bis 800 nm, bevorzugt 50 bis 600 nm. Da die Schicht (A) im wesentlichen den Farbton der erfindungsgemäßen Pigmente bestimmt, hat sie für ein besonders ausgeprägtes Farbenspiel zeigende und daher auch bevorzugte Glanzpigmente, die nur ein Schichtpaket (A) + (B) aufweisen, eine Mindestschichtdicke von etwa 100 nm.

Mit wachsender Schichtdicke von (A) durchläuft man bei den mit der Schicht (A) und der metallischen Schicht (B) belegten Pigmenten bei einem Betrachtungswinkel von 25° mehrmals nacheinander die Interferenzfarben blau, grün, gold, rot. Die Winkelabhängigkeit des Farbtons nimmt von der ersten Interferenzfarbenserie nach höheren Serien (also dicker werdenden Schichten (A)) zu. Die zweite Interferenzfarbenserie beginnt bei einer Schichtdicke von etwa 275 nm und die dritte Serie bei etwa 450 nm. Die Interferenzfarben der dritten Serie sind extrem winkelabhängig. So zeigt z.B. ein in der direkten Aufsicht grün reflektierendes Pigment bei zunehmender Schrägsicht einen Farbwechsel über blau nach rot.

Die absorbierende Schicht (B) besteht vorzugsweise im wesentlichen aus elementarem Silicium oder Gemischen von elementarem Silicium und Metallen, die sich durch Gasphasenzersetzung der Carbonyle abscheiden lassen, wie Molybdän, Chrom, Wolfram und Eisen. Diese Gemische enthalten in der Regel auch die jeweiligen Silicide. Besonders bevorzugt sind Gemische von Silicium und Molybdän, die üblicherweise Molybdänsilicid MoSi₂ enthalten. Im allgemeinen beträgt der Siliciumgehalt der gemischten Schichten (B) etwa 5 bis 90 Gew.-%, bevorzugt 8 bis 80 Gew.-% und besonders bevorzugt 30 bis 60 Gew.-%. Derartige Schichten (B) zeichnen sich durch hohe Echtheiten, vor allem auch hohe Schwitzwasserbeständigkeit, aus.

Die Schicht (B) sollte für sichtbares Licht zumindest teilweise durchlässig (semitransparent) sein und hat daher in der Regel eine Schichtdicke von etwa 5 bis 30 nm. Für Schichten (B), die Silicium und Molybdän enthalten, sind dabei Schichtdicken von etwa 5 bis 20 nm und für "reine" Siliciumschichten (B) Schichtdicken von 20 bis 30 nm bevorzugt.

Sind mehrere (z.B. 2, 3 oder 4) Schichtpakete (A) + (B) enthalten, dann ist die Schicht (A) insbesondere 20 bis 400 nm dick und die Schicht (B) bevorzugt 5 bis 10 nm dick. Bevorzugt sind jedoch die Glanzpigmente mit nur einem Schichtpaket (A) + (B).

Die erfindungsgemäßen Glanzpigmente können noch eine äußere Schicht (C) aufweisen, die eine zusätzliche Variation der Pigmentkoloristik möglich macht.

Die Schicht (C) ist vorzugsweise aus hochbrechenden Metalloxiden, die sowohl farblos als auch selektiv absorbierend sein können, aufgebaut. Beispiele für bevorzugte Metalloxide sind Titandioxid, Zirkonoxid, Eisen(III)oxid und Chrom(III)oxid.

Die Dicke der Schicht (C) beträgt im allgemeinen etwa 1 bis 400 nm, vorzugsweise 5 bis 250 nm. Dabei sind z.B. für Fe₂O₃- und Cr₂O₃-haltige Schichten (C) Schichtdicken von 1 bis 20 nm und für TiO₂- und ZrO₂-haltige Schichten (C) Schichtdicken von bis zu 100 nm bevorzugt.

Die Schicht (C) trägt zur Interferenz des Pigmentes bei und setzt die Interferenzreihe an der durch das mit (A) und (B) beschichtete Substrat bestimmten Stelle fort. Farbige Metalloxide wie Eisen- und Chromoxid werden die Interferenzfarbe des Mehrschichtsystems durch Beimischen ihrer Absorptionsfarbe modifizieren und mit zunehmender Schichtdicke schließlich überdecken.

Insgesamt zeichnen sich bei den erfindungsgemäßen Glanzpigmenten alle Schichten durch ihren gleichmäßigen, homogenen und filmartigen Aufbau und ihre Fähigkeit zur Interferenz auch der dickeren Schichten aus, so daß kräftige Interferenzfarben zeigende Mehrschichtsysteme entstehen, bei denen die Substratteilchen an allen Seiten und nicht nur an der Plättchenober- und -unterseite beschichtet sind.

Aus koloristischen Gründen sind auch Mischungen der erfindungsgemäßen metallischen Pigmente (I) mit ebenfalls mehrfach beschichteten silikatischen Plättchen (II) von besonderem Interesse.

Als silikatische Substrate kommen dabei insbesondere helle bzw. weiße Glimmer in Betracht, wobei Schuppen von vorzugsweise naß vermahlenem Muskovit besonders bevorzugt sind. Selbstverständlich sind auch andere natürliche Glimmer wie Phlogopit und Biotit, künstliche Glimmer, Talk- und Glasschuppen geeignet.

Die zum Einsatz kommenden silikatischen Substratteilchen weisen eine Metalloxidschicht (A') auf, die vorzugsweise aus hochbrechenden Metalloxiden wie Titan-, Zirkon-, Zink-, Zinn-, Chrom-, Eisenoxid und/oder Bismutoxychlorid aufgebaut ist. Aluminium- und Siliciumoxid können ebenfalls enthalten sein.

Besonders bevorzugt sind Glimmerpigmente, die eine im wesentlichen aus Titandioxid bestehende und die weiteren genannten Oxide höchstens in untergeordneter Menge enthaltende Schicht (A') aufweisen.

Metalloxidbeschichtete silikatische Pigmente sind allgemein bekannt und auch unter den Bezeichnungen Iriodin® (Merck, Darmstadt), Flonac® (Kemira Oy, Pori) oder Mearlin® (Mearl Corporation, New York) im Handel.

Durch geeignete Wahl der silikatischen Pigmente (II) kann das Farbenspiel der Metallpigmente (I) variiert oder ergänzt werden.

Zeigt beispielsweise ein mit (A) und (B) beschichtetes metallisches Substrat bei einem Betrachtungswinkel von 25° einen goldenen Farbton, so kann dieser durch Beimischen eines eine rotgoldene Interferenzfarbe aufweisenden titandioxidbeschichteten Glimmerpigments zu dem nur mit (A) beschichteten Metallpigment und anschließende gemeinsame Beschichtung mit (B) in Richtung auf einen rötlicheren Farbton verschoben werden.

Die Zusammensetzung der erfindungsgemäßen Glanzpigmentmischungen wird von der gewünschten Koloristik bestimmt.

Prinzipiell kann das Gewichtsverhältnis metallisches Pigment (I): silikatisches Pigment (II) von 1:99 bis 99:1 variiert werden. Um ein ausreichendes Deckvermögen zu erreichen, enthalten die erfindungsgemäßen Pigmentmischungen vorzugsweise mindestens 5 Gew.-% metallisches Glanzpigment (I).

Der bevorzugte Weg zur Herstellung der erfindungsgemäßen Pigmentmischungen ist die gemeinsame Beschichtung der bereits gemäß Schritt (a) mit der Schicht (A) und mit einer Schicht (A') belegten Substratteilchen mit der Schicht (B) und gewünschtenfalls der Deckschicht (C).

Selbstverständlich können jedoch auch alle Schichten getrennt aufgebracht werden und die beschichteten Pigmente dann anschließend gemischt werden. Bei dieser Vorgehensweise können die Schichten (B) und (B') sowie (C) und (C') zusätzlich variiert werden.

Bei dem erfindungsgemäßen Verfahren zur Herstellung der Glanzpigmente werden die einzelnen Schichten durch Gasphasenzersetzung geeigneter flüchtiger Metallverbindungen (chemical vapor deposition, CVD) oder naßchemisch durch hydrolytische Zersetzung insbesondere organischer Metallverbindungen aufgebracht.

Selbstverständlich können beide Vorgehensweisen beliebig zur Herstellung der einzelnen Schichten kombiniert werden.

Zur Erzeugung der Silicium- und/oder Aluminiumoxidschichten (A) sind die naßchemische und die CVD-Verfahrensvariante gleichermaßen geeignet.

Bei der naßchemischen Variante, die in der DE-A-44 05 492 beschrieben ist, werden organische Silicium- und/oder Aluminiumverbindungen, bei denen die organischen Reste über Sauerstoffatome an die Metalle gebunden sind, in Gegenwart der Substratteilchen und eines organischen Lösungsmittels, in welchem die Metallverbindungen löslich sind und welches mit Wasser mischbar ist, hydrolysiert.

Die bevorzugte Ausführungsform besteht dabei in der Hydrolyse der Metallalkoholate (insbesondere Tetraethoxysilan und Aluminiumtriisopropanolat) in Gegenwart eines Alkohols (insbesondere Isopropanol) und von wäßrigem Ammoniak als Katalysator.

Verfahrenstechnisch geht man vorzugsweise so vor, daß man Substratteilchen, Isopropanol, Wasser und Ammoniak vorlegt, diese Mischung unter Rühren auf 40°C bis 80°C, insbesondere etwa 60°C bis 70°C, erhitzt und eine Lösung des Metallalkoholats in Isopropanol kontinuierlich zudosiert. Nach einer Nachrührzeit von meist etwa 1 bis 15 h kühlt man die Mischung auf Raumtemperatur ab und isoliert das beschichtete Pigment durch Abfiltrieren, Waschen und Trocknen.

Bei der CVD-Variante, die in der nicht vorveröffentlichten DE-A-44 37 752 beschrieben ist, werden Silane, die mindestens einen Alkanoyloxyrest enthalten, in der Gasphase mit Wasserdampf und, wenn die Silane auch Alkyl- oder Phenylreste aufweisen, Sauerstoff in Gegenwart bewegter Substratteilchen zersetzt.

Bevorzugte Silane weisen dabei Alkoxy- und Alkanoyloxyreste auf, besonders bevorzugt ist Di-tert.-butoxydiacetoxysilan.

Zur Durchführung der CVD-Variante empfiehlt sich wie allgemein für CVD-Verfahren üblich die Verwendung eines Wirbelschichtreaktors, wie er beispielsweise in der EP-A 45 851 beschrieben ist. Die Substratteilchen werden im Reaktor unter Fluidisierung (Verwirbelung) mit einem inerten Wirbelgas wie Stickstoff auf die gewünschte Reaktionstemperatur (in der Regel 100 bis 600°C, bevorzugt 150 bis 300°C) erhitzt, Silan und Wasserdampf (und/oder Sauerstoff) werden dann mit Hilfe inerter Trägergasströme (vorteilhaft Teilströmen des Wirbelgases) aus vorgeschalteten Verdampfergefäßen über getrennte Düsen eingeleitet, wobei die Silankonzentration zweckmäßigerweise bei ≤ 5 Vol.-%, vorzugsweise ≤ 2 Vol.-%, bezogen auf die Gesamtgasmenge im Reaktor, gehalten wird. Die Wasserdampfmenge sollte mindestens der stöchiometrisch zur Hydrolyse des Silans erforderlichen Menge entsprechen, vorzuziehen ist jedoch die 10 bis 100fache Menge.

Die elementares Silicium enthaltenden Schichten (B) werden beim erfindungsgemäßen Herstellungsverfahren durch Gasphasenzersetzung von Silanen aufgebracht. Sollen die Schichten (B) neben Silicium auch Metalle enthalten, so werden die Silane gleichzeitig mit den entsprechenden Metallcarbonylen zersetzt.

Geeignet sind hierfür Silane der Formel I

SiₙX₂ₙ₊₂ I

in der n für 1 bis 3 steht und X für den Fall n=1 Wasserstoff oder gleiche oder verschiedene C₁-C₃-Alkylreste, wobei mindestens ein Rest X Wasserstoff ist, und für den Fall n>1 Wasserstoff bedeutet.

Neben Mono-, Di- und Trialkylsilanen wie Dimethylsilan eignen sich insbesondere die rein wasserstoffhaltigen Silane Monosilan (SiH₄), Trisilan (Si₃H₈) und bevorzugt Disilan (Si₂H₆).

Verfahrenstechnisch geht man beim Aufbringen der elementares Silicium enthaltenden Schichten (B) zweckmäßigerweise so vor, daß man das Silan aus einer Druckgasflasche zusammen mit einem Inertgasstrom (insbesondere Argon) über eine vorzugsweise temperierte Düse in den Beschichtungsreaktor überführt und dort bei in der Regel 100 bis 600°C, bevorzugt 150 bis 500°C, thermisch zersetzt, wobei sich auf den im Reaktor bewegten, mit der Schicht (A) belegten Substraten ein Siliciumfilm abscheidet. Die Gasmenge Silan sollte hierbei im allgemeinen nicht mehr als 2 Vol.-%, vorzugsweise nicht mehr als 1 Vol.-%, der Gesamtgasmenge im Reaktor betragen.

Sollen die Schichten (B) aus Gemischen von elementarem Silicium und Metallen bestehen, so führt man über eine zweite Reaktoröffnung einen weiteren Inertgasstrom ein, der vorher über eine Verdampfervorlage mit dem entsprechenden Metallcarbonyl geleitet und auf diese Weise mit Metallcarbonyldampf beladen wurde. Metallcarbonyl und Silan zersetzen sich dann gleichzeitig im Reaktor und scheiden sich als gemischte Silicium/Metall-Schicht bzw. als Metallsilicidfilm auf den Substratteilchen ab.

Als Reaktor wird insbesondere der oben erwähnte Wirbelschichtreaktor bevorzugt, man kann jedoch auch einen Einhalsrundkolben aus Quarzglas verwenden, der über einen Motor gedreht wird, mit Gaszu- und -ableitungen in der Drehachse versehen ist und von einem zweischaligen Klappofen beheizt wird (Drehkugelofen).

Im Prinzip läßt sich jeder beheizbare Mischer, der die Substratteilchen mittels entsprechender Einbauten schonend bewegt und eine Gaszu- und -ableitung gestattet, als Reaktor einsetzen.

Für eine kontinuierliche Verfahrensführung in technischem Maßstab eignet sich z.B. auch ein Drehrohrofen, dem die Substratteilchen und die Silan/Inertgas-Mischung (und Metallcarbonyl/Inertgas-Mischung) fortlaufend zugeführt werden.

Die äußeren Schichten (C) werden beim erfindungsgemäßen Verfahren durch hinlänglich bekannte oxidative Gasphasenzersetzung der Metallcarbonyle (z.B. Eisenpentacarbonyl, Chromhexacarbonyl) bzw. hydrolytische Gasphasenzersetzung der Metallalkoholate (z.B. Titan- und Zirkon-tetra-n- und -isopropanolat) (EP-A-33 457, EP-A-338 428) aufgebracht.

Mit Hilfe des erfindungsgemäßen Herstellungsverfahrens können die mehrfach beschichteten Glanzpigmente in einfacher Weise in großen Mengen reproduzierbar hergestellt werden. Es werden vollständig umhüllte Pigmentteilchen mit hoher Qualität der einzelnen Beschichtungen (homogen, filmartig) erhalten.

Die erfindungsgemäßen Glanzpigmente und Glanzpigmentmischungen eignen sich vorteilhaft für viele Zwecke, wie zur Einfärbung von Kunststoffen, Gläsern, keramischen Produkten, Zubereitungen der dekorativen Kosmetik und besonders von Lacken, insbesondere auch Automobillacken, Tinten und Druckfarben, vor allem Sicherheitsdruckfarben. Bei der Applikation im Druck sind alle industrieüblichen Druckverfahren, z.B. Siebdruck, Tiefdruck, Bronzierdruck, Flexodruck und Offsetdruck, geeignet.

Für diese Anwendungszwecke lassen sich die erfindungsgemäßen Pigmente auch vorteilhaft in Abmischung mit transparenten und deckenden Weiß-, Bunt- und Schwarzpigmenten sowie auch herkömmlichen transparenten, bunten und schwarzen Glanzpigmenten auf der Basis von metalloxidbeschichteten Glimmer- und Metallpigmenten, plättchenförmigen Eisenoxiden, Graphit, Molybdänsulfid und plättchenförmigen organischen Pigmenten verwenden.

### Beispiele

### Herstellung und Anwendung von erfindungsgemäßen Glanzpigmenten

Bei der Einarbeitung der Pigmente in Lack wurden jeweils 0,4 g Pigment in 3,6 g eines Polyester-Mischlackes mit 21 Gew.-% Feststoffanteil eingerührt und 2 min im Red Devil dispergiert. Mit einer Rakel (160 µm Naßfilmdicke) wurden auf schwarzweißem Karton Abzüge der pigmentierten Lacke angefertigt. Zur Prüfung der Wasserbeständigkeit der Lackierungen wurden die Lacke auf transparente Polyesterfolien aufgerakelt und über längere Zeit in Wasser eingetaucht.

Bei der Anwendung der Pigmente im Siebdruck wurden 10 g Pigment in 90 g einer handelsüblichen Bindemittellösung (22,5 g PVC-Mischpolymerisat Laroflex® MP45, 4,5 g Methoxypropylacetat, 13,5 g n-Hexyldiglykol, 49,5 g Butylglykol) eingerührt. Die so hergestellte Siebdruckfarbe wurde mit einer handelsüblichen Siebdruckmaschine (Siebmaschenweite 112 bis 150 µm) auf gestrichenes, titandioxidbeschichtetes Papier in einer Schichtdicke von 45 µm aufgebracht und an der Luft getrocknet.

### Beispiel 1

a) In einem mit Rückflußkühler und Rührapparatur versehenen Rundkolben wurden 100 g feinteiliges Aluminiumpulver (mittlerer Teilchendurchmesser 20 µm) in 1,5 l Isopropanol aufgeschlämmt. Nach Zugabe von 400 ml Wasser und 40 ml einer 25 gew.-%igen wäßrigen Ammoniaklösung wurde die Suspension unter kräftigem Rühren auf 65°C erhitzt. Gleichzeitig wurde mit der Zudosierung eines Gemisches von 600 ml Isopropanol und 600 g Tetraethoxysilan begonnen (Dosiergeschwindigkeit 100 ml/h, 12 h). Nach einer Nachrührzeit von 10 h und Abkühlen der Suspension wurde das Produkt abfiltriert, gründlich mit Isopropanol gewaschen und bei 80°C getrocknet.
   Das beschichtete Aluminiumpulver hatte einen Siliciumgehalt von 26,7 Gew.-% und zeigte einen schwachen Grünstich.
b) Anschließend wurden 50 g des beschichteten Aluminiumpulvers im oben beschriebenen Drehkugelreaktor nach zweistündigem Inertisieren mit 20 l/h Argon auf 500°C erhitzt. Dann wurden 8,5 l/h Disilan aus einer Druckgasflasche über eine auf 300°C temperierte Düse im Gemisch mit Argon (10 Vol.-% Disilan im Gasgemisch) in den Reaktor überführt und dort zu Silicium und Wasserstoff zersetzt. Nach 1 h wurde die Silanzufuhr beendet. Der Reaktor wurde noch 2 h bei 500°C gehalten, dann auf Raumtemperatur abgekühlt und belüftet.

Das erhaltene Pigment hatte einen Siliciumgehalt von insgesamt 28,8 Gew.-%, was einem Anteil der Siliciumbeschichtung (B) am Pigment von 2,1 Gew.-% entspricht.

Das Pigment zeigte bei Applikation im Lack und im Siebdruck bei starkem metallischen Glanz eine intensiv grüngoldene Interferenzfarbe, die bei steileren Betrachtungswinkeln weiter ins Grünstichige abkippte. Eine mit einem kommerziellen Farbkopierer (Canon CLC 500) hergestellte Farbkopie des Siebdrucks zeigte kein winkelabhängiges Farbenspiel mehr, sondern lediglich eine Mischfarbe.

### Beispiel 2

a) In der Apparatur aus Beispiel 1 wurden 200 g des feinteiligen Aluminiumpulvers in 1 1 Isopropanol aufgeschlämmt. Unter kräftigem Rühren wurde die Suspension auf 80°C erhitzt. Gleichzeitig wurde mit der Zudosierung von 1350 g Tetraethoxysilan und (parallel dazu) eines Gemisches von 860 ml Wasser, 86 ml einer 25 gew.-%igen wäßrigen Ammoniaklösung und 400 ml Isopropanol begonnen. Die Dosiergeschwindigkeit betrug in beiden Fällen 100 ml/h, die Dosierung war in 14 h beendet. Nach einer Nachrührzeit von 10 h wurde wie in Beispiel 1 aufgearbeitet.
   Das beschichtete Aluminiumpulver hatte einen Siliciumgehalt von 28,2 Gew.-% und zeigte einen schwachgrünen Farbschimmer.
b) Anschließend wurden 30 g des beschichteten Aluminiumpulvers im wie in Beispiel 1 inertisierten Drehkugelofen auf 450°C erhitzt. Dann wurden über einen Zeitraum von 7 h 10 l/h Disilan über eine auf 100°C temperierte Düse im Gemisch mit Argon (10 Vol.-% Disilan im Gasgemisch) und gleichzeitig (ebenfalls in 7 h) über eine weitere Zuleitung 5 g Molybdänhexacarbonyl aus einer auf 40°C temperierten Verdampfervorlage mit einem Stickstoffstrom von 20 l/h in den Reaktor überführt und dort zu einem Silicium/Molybdän-Gemisch, Wasserstoff und CO zersetzt. Der Reaktor wurde noch 2 h bei 450°C gehalten, dann auf Raumtemperatur abgekühlt und belüftet.

Das erhaltene Pigment hatte einen Molybdängehalt von 1,3 Gew.-% und einen Siliciumgehalt von insgesamt 32,8 Gew.-%, was einem Siliciumanteil der Schicht (B) am Pigment von 5,0 Gew.-% entspricht.

Das Pigment zeigte bei Applikation im Lack und im Siebdruck bei starkem metallischen Glanz eine intensiv grüne Interferenzfarbe, die bei steileren Betrachtungswinkeln nach Rot abkippte.

### Beispiel 3

200 g des SiO₂-beschichteten Aluminiumpigments aus Beispiel 2a) wurden im Wirbelschichtreaktor unter Fluidisierung mit 600 l/h Stickstoff auf 300°C erhitzt. Über eine der seitlich angebrachten Düsen wurde über einen Zeitraum von 7 h ein Gasgemisch aus 0,3 l/h Disilan, 2,7 l/h Argon und 200 l/h Stickstoff eingeleitet. Über die andere seitliche Düse wurden gleichzeitig in ebenfalls 7 h 25 g Molybdänhexacarbonyl mit einem Stickstoffstrom von 400 l/h aus einer auf 70°C temperierten Vorlage in den Reaktor überführt. Anschließend wurde der Reaktor noch 2 h bei 300°C gehalten, dann unter weiterer Einleitung von 600 l/h Stickstoff auf Raumtemperatur abgekühlt und belüftet.

Das erhaltene Pigment hatte einen Molybdängehalt von 3,4 Gew.-% und einen Siliciumgehalt von insgesamt 27,5 Gew.-%, was einem Siliciumanteil der Schicht (B) am Pigment von 0,3 Gew.-% entspricht.

Das Pigment zeigte bei Applikation im Lack und im Siebdruck bei starkem metallischen Glanz eine intensiv grüne Interferenzfarbe, die bei steileren Betrachtungswinkeln nach Rot abkippte.

### Vergleichsbeispiel

180 g des SiO₂-beschichteten Aluminiumpigments aus Beispiel 2a) wurden analog Beispiel 3, jedoch ohne Zufuhr von Silan mit Molybdän beschichtet.

Das erhaltene Pigment zeigte bei Applikation eine intensiv blaue Interferenzfarbe, die bei steileren Betrachtungswinkeln nach Rot abkippte.

Bei der Prüfung der Wasserbeständigkeit wurde eine dieses Pigment enthaltende Lackierung bereits über Nacht entfärbt, während Lackierungen, welche die Pigmente der Beispiele 1 bis 3 enthielten, auch nach mehreren Tagen noch keine Entfärbung zeigten.

## Patentansprüche

1. Goniochromatische Glanzpigmente auf der Basis von mehrfach beschichteten, plättchenförmigen, metallischen Substraten, die mindestens ein Schichtpaket aus
A) einer im wesentlichen aus Siliciumoxid, Siliciumoxidhydrat, Aluminiumoxid und/oder Aluminiumoxidhydrat bestehenden Schicht und
B) einer nichtselektiv absorbierenden, für sichtbares Licht zumindest teilweise durchlässigen, elementares Silicium enthaltenden Schicht
sowie gewünschtenfalls zusätzlich
C) eine äußere Schicht, die im wesentlichen aus farblosem oder selektiv absorbierendem Metalloxid besteht,
aufweisen.

2. Glanzpigmente nach Anspruch 1, bei denen die Schicht (B) im wesentlichen aus elementarem Silicium oder aus Gemischen von elementarem Silicium, Metallen, die sich durch Gasphasenzersetzung ihrer Carbonyle abscheiden lassen, und/oder den Metallsiliciden besteht.

3. Glanzpigmente nach Anspruch 1 oder 2, bei denen die Schicht (B) im wesentlichen aus elementarem Silicium, aus Gemischen von elementarem Silicium mit Molybdän, Chrom, Wolfram und/oder Eisen und/oder den entsprechenden Metallsiliciden besteht.

4. Glanzpigmente nach den Ansprüchen 1 bis 3, bei denen die Schicht (C) Titandioxid, Zirkondioxid, Eisen(III)-oxid und/oder Chrom(III)oxid enthält.

5. Glanzpigmente nach den Ansprüchen 1 bis 4, die nur ein Schichtpaket (A) + (B) enthalten.

6. Glanzpigmente nach den Ansprüchen 1 bis 5, bei denen das metallische Substrat im wesentlichen aus Aluminiumplättchen besteht, die nach gängigen Verdüsungs- und Mahltechniken hergestellt worden sind.

7. Glanzpigmente nach den Ansprüchen 1 bis 6, bei denen das metallische Substrat im wesentlichen aus Aluminiumplättchen, die mit einer ferromagnetischen Schicht belegt und/oder passiviert sein können, besteht.

8. Glanzpigmentmischungen aus
I) den Glanzpigmenten gemäß den Ansprüchen 1 bis 7 und
II) mehrfach beschichteten silikatischen Plättchen, die
A') eine im wesentlichen aus farblosem oder selektiv absorbierendem Metalloxid bestehende Schicht,
B') eine nichtselektiv absorbierende metallische, für sichtbares Licht zumindest teilweise durchlässige, elementares Silicium enthaltende Schicht und gewünschtenfalls
C') eine äußere Schicht, die im wesentlichen aus farblosem oder selektiv absorbierendem Metalloxid besteht,
aufweisen,
als wesentlichen Komponenten.

9. Verfahren zur Herstellung von Glanzpigmenten gemäß den Ansprüchen 1 bis 8, dadurch gekennzeichnet, daß man
die Schichten (A) durch Gasphasenzersetzung flüchtiger Siliciumorganyle mit Wasserdampf und/oder Sauerstoff oder durch hydrolytische Zersetzung organischer Silicium- oder Aluminiumverbindungen, bei denen die organischen Reste über Sauerstoffatome an das Metall gebunden sind, in Gegenwart eines organischen Lösungsmittels, in welchem die Metallverbindungen löslich sind und welches mit Wasser mischbar ist, und anschließende Trocknung,
die Schichten (B) durch Gasphasenzersetzung von Silanen der Formel I
SiₙX₂ₙ₊₂ I
in der die Variablen folgende Bedeutung haben:
n 1 bis 3;
X für den Fall n=1 Wasserstoff oder gleiche oder verschiedene C₁-C₃-Alkylreste, wobei mindestens ein Rest X Wasserstoff bedeutet, und
für den Fall n>1 Wasserstoff,
und gegebenenfalls Metallcarbonylen in einer inerten Atmosphäre und gewünschtenfalls
die Schicht (C) durch Gasphasenzersetzung flüchtiger Metallverbindungen in Gegenwart von Sauerstoff und/oder Wasserdampf auf die metallischen Substratplättchen aufbringt.

10. Verwendung von Glanzpigmenten gemäß den Ansprüchen 1 bis 8 zur Einfärbung von Lacken, Druckfarben, Tinten, Kunststoffen, Gläsern, keramischen Produkten und Zubereitungen der dekorativen Kosmetik.

## Claims

1. Goniochromatic luster pigments based on multiply coated platelet-shaped metallic substrates comprising at least one layer packet of
A) a layer consisting essentially of silicon oxide, silicon oxide hydrate, aluminum oxide and/or aluminum oxide hydrate, and
B) a nonselectively absorbing, elemental-silicon-containing layer which is at least partially transparent to visible light,
and also, if desired, additionally
C) an outer layer which consists essentially of colorless or selectively absorbing metal oxide.

2. Luster pigments as claimed in claim 1 wherein layer (B) consists essentially of elemental silicon or of mixtures of elemental silicon, metals which are depositable by gas phase decomposition of their carbonyls, and/or the metal silicides.

3. Luster pigments as claimed in claim 1 or 2 wherein layer (B) consists essentially of elemental silicon, of mixtures of elemental silicon with molybdenum, chromium, tungsten and/or iron and/or the corresponding metal silicides.

4. Luster pigments as claimed in any of claims 1 to 3 wherein layer (C) comprises titanium dioxide, zirconium dioxide, iron(III) oxide and/or chromium(III) oxide.

5. Luster pigments as claimed in any of claims 1 to 4 comprising only one layer packet (A) + (B).

6. Luster pigments as claimed in any of claims 1 to 5 wherein the metallic substrate consists essentially of aluminum platelets produced by common atomizing and grinding techniques.

7. Luster pigments as claimed in any of claims 1 to 6 wherein the metallic substrate consists essentially of aluminum platelets which may be coated with a ferromagnetic layer and/or passivated.

8. Luster pigment mixtures of
I) the luster pigments of any of claims 1 to 7, and
II) multiply coated silicatic platelets comprising
A') a layer consisting essentially of colorless or selectively absorbing metal oxide,
B') a nonselectively absorbing, elemental-silicon-containing, metallic layer which is at least partially transparent to visible light, and if desired
C') an outer layer which consists essentially of colorless or selectively absorbing metal oxide,
as essential components.

9. A process for producing luster pigments as claimed in any of claims 1 to 8, which comprises coating the metallic substrate platelets with
layers (A) by gas phase decomposition of volatile organosilicons using water vapor and/or oxygen or by hydrolytic decomposition of organic silicon or aluminum compounds in which the organic radicals are attached to the metal via oxygen atoms in the presence of an organic solvent in which the metal compounds are soluble and which is miscible with water, and subsequent drying,
layers (B) by gas phase decomposition of silanes of the formula I
SiₙX₂ₙ₊₂ I
where
n is from 1 to 3, and
X is hydrogen or identical or different C₁-C₃-alkyl when n=1, at least one X being hydrogen, or is hydrogen when n>1,
with or without metal carbonyls in an inert atmosphere and if desired
layer (C) by gas phase decomposition of volatile metal compounds in the presence of oxygen and/or water vapor.

10. The use of luster pigments as claimed in any of claims 1 to 8 for coloring paints, inks, including printing inks, plastics, glasses, ceramic products and decorative cosmetic preparations.

## Revendications

1. Pigments brillants goniochromatiques à base de substrats métalliques sous forme de paillettes plusieurs fois revêtues, présentant au moins un système de couches formé
A) d'une couche constituée essentiellement d'oxyde de silicium, d'oxyde de silicium hydraté, d'oxyde d'aluminium et/ou d'oxyde d'aluminium hydraté et
B) d'une couche absorbant de façon non sélective, au moins partiellement transparente pour la lumière visible, et contenant du silicium élémentaire,
ainsi qu'éventuellement en outre
C) une couche extérieure constituée pour l'essentiel d'oxyde métallique incolore ou à absorption sélective.

2. Pigments brillants selon la revendication 1, pour lesquels la couche (B) est constituée pour l'essentiel de silicium élémentaire ou de mélanges de silicium élémentaire, de métaux qui sont séparables par décomposition en phase gazeuse de leur carbonyle, et/ou de siliciures de métaux.

3. Pigments brillants selon la revendication 1 ou 2, pour lesquels la couche (B) est essentiellement constituée de silicium élémentaire, de mélanges de silicium élémentaire avec du molybdène, du chrome, du tungstène et/ou du fer et/ou les siliciures de métaux correspondants.

4. Pigments brillants selon l'une quelconque des revendications 1 à 3, pour lesquels la couche (C) contient du dioxyde de titane, du dioxyde de zirconium, de l'oxyde de fer (III) et/ou de l'oxyde de chrome (III).

5. Pigments brillants selon l'une quelconque des revendications 1 à 4, ne contenant qu'un système de couches (A) + (B).

6. Pigments brillants selon l'une quelconque des revendications 1 à 5, pour lesquels le substrat métallique est essentiellement constitué de paillettes d'aluminium fabriquées selon des techniques usuelles d'atomisation et de broyage.

7. Pigments brillants selon l'une quelconque des revendications 1 à 6, pour lesquels le substrat métallique est constitué pour l'essentiel de paillettes d'aluminium qui peuvent être revêtues d'une couche ferromagnétique et/ou passivées.

8. Mélanges de pigments brillants formés
I) des pigments brillants selon l'une quelconque des revendications 1 à 7 et
II) de paillettes silicatées à revêtements multiples, qui présentent
A') une couche constituée essentiellement d'un oxyde métallique incolore ou à absorption sélective,
B') une couche métallique absorbant de façon non sélective, au moins partiellement transparente pour la lumière visible, et contenant du silicium élémentaire et éventuellement
C') une couche extérieure constituée essentiellement d'un oxyde métallique incolore ou à absorption sélective,
en tant que composants essentiels.

9. Procédé de préparation de pigments brillants selon l'une quelconque des revendications 1 à 8, caractérisé en ce que l'on dépose sur les paillettes de substrat métallique
les couches (A) par décomposition en phase gazeuse de composés organiques et volatils du silicium avec de la vapeur d'eau et/ou de l'oxygène ou par décomposition hydrolytique de composés organiques du silicium ou de l'aluminium, pour lesquels les restes organiques sont liés au métal par des atomes d'oxygène, en présence d'un solvant organique, dans lequel les composés métalliques sont solubles et qui est miscible à l'eau, et séchage subséquent,
les couches (B) par décomposition en phase gazeuse de silanes de formule I
SiₙX₂ₙ₊₂ I
dans laquelle les variables ont la signification suivante :
n vaut de 1 à 3,
X représente lorsque n = 1, des atomes d'hydrogène ou des restes alkyle en C₁-C₃ identiques ou différents, où au moins un reste X représente un atome d'hydrogène, et
lorsque n > 1, des atomes d'hydrogène,
et éventuellement des carbonyles métalliques dans une atmosphère inerte et éventuellement
les couches (C) par décomposition en phase gazeuse de composés métalliques volatils en présence d'oxygène et/ou de vapeur d'eau.

10. Utilisation de pigments brillants selon l'une quelconque des revendications 1 à 8 pour la coloration de vernis, d'encres d'imprimerie, de teintures, de matières plastiques, de verres, de produits céramiques et de préparations pour la cosmétique décorative.
